# EUROPEAN PATENT APPLICATION

(11) **EP 2 921 175 A1**
(43) Date of publication of application: **23.09.2015**
(21) Application number: 14160731.7
(22) Date of filing: 19.03.2014
(51) Int. Cl.: A61K 36/185, A61K 36/28, A61K 36/738, A61K 41/00, A61P 31/12, A61P 11/00, A61K 31/17, A61K 33/04

(54) **Herbal extracts having anti-recurrent respiratory papillomatosis properties**

(71) Applicant: RosePharmed, 14667 93143 Tehran (IR)
(72) Inventor: Garibdoust, Farhad, Tehran (IR); Khorramkhorshid, Hamid Reza, Tehran (IR); Radmanesh, Ramin, Tehran (IR); Farzamfar, Bardia, Tehran (IR); Farhadi, Mohammad., Tehran (IR); Madani, Hessameddin., Tehran (IR); Kamali, Koorosh., Tehran (IR)
(74) Representative: Scholz, Volker

(57) **Abstract**

The present invention relates to a herbal extract obtainable by a method, comprising the following steps: (a) providing a plant material derived from *Rosa sp., Urtica dioica* and/or *Tanacetum vulagare;* (b) drying a plant material; (c) adding an organic solvent; (d) incubating the mixture of plant material and organic solvent; (e) obtaining the herbal extract; (f1) adding selenium and/or an organic or inorganic salt thereof; and/or (f2) adding urea; and (g) exposing the herbal extract to a pulsed electromagnetic field, for use in the treatment of Recurrent Respiratory Papillomatosis.

## Description

The present invention refers to a herbal extract for use in the treatment of Recurrent Respiratory Papillomatosis.

### Background of the invention

Recurrent Respiratory Papillomatosis (RRP), formerly known as juvenile laryngeal papillomatosis or glottal papillomatosis, is the most common benign tumor of the respiratory tract in children and adolescent. The cause of the disease is Human papilloma virus (HPV) types 6 and 11 for which there is, unfortunately, no definitive cure. The frequency of RRP occurrence in children and adult is 1-4/100,000 and 1.8-3.9/100,000 persons, respectively. RRP is most often located in the larynx. The most typical symptoms are hoarseness and sever stridor and dyspnea in progressive cases. Diagnosis of the disease involves physical examination, bronchoscopy, histopathologic detection and PCR.

The mainstay of treatment for RRP is surgical removing of the lesions by different methods, including physical debridement, CO₂ laser vaporization, and a newer method employing the laryngeal microdebrider. The clinical course is not predictable but usually infected patients typically require repeat surgical excisions due to hoarseness or respiratory obstruction signs every 3-4 years.

Patient's immune system seems to have main role in infection pathogenesis and spontaneous remission. Thus, a number of adjuvant therapies have been tried to control aggressive papillomatosis. These therapies include topical chemotherapy, steroids, cidofovir, cis retinoic acid, podophyllin, autogenous vaccine, immune stimulators, acyclovir, accutane, and interferon.

Although interferon therapy has shown significant problems, it is considered to be the most successful treatment (30-50% eradication & 70-80% partial response).

Thus, it is an object of the present invention to provide a pharmaceutically active composition for use in the treatment of Recurrent Respiratory Papillomatosis which overcomes drawbacks of the prior art, particularly a composition being safe and effective in the treatment of RRP. It is also an object of the present invention to provide a pharmaceutically active composition for use in the treatment of RRP which can advantageously be used in combination with classic surgical operation.

### Summary of the invention

This object has been solved by a herbal extract obtainable by a method, comprising the following steps: (a) providing a plant material derived from *Rosa sp., Urtica dioica* and/or *Tanacetum vulagare;* (b) drying a plant material; (c) adding an organic solvent; (d) incubating the mixture of plant material and organic solvent; (e) obtaining the herbal extract; (f1) adding selenium and/or an organic or inorganic salt thereof; and/or (f2) adding urea; and (g) exposing the herbal extract to a pulsed electromagnetic field, for use in the treatment of Recurrent Respiratory Papillomatosis.

The plant material to be extracted may be provided separately or in any mixed combination. Preferably, the plant material of *Urtica dioica* and *Tanacetum vulagare* is extracted jointly, with the plant material of *Rosa sp.* is extracted separately. After respective extracts are obtained, these may be combined.

It is also preferred that the extract of *Rosa sp.* is exposed to a pulsed electromagnetic field before being combined with the other extract(s).

In a preferred embodiment, the electromagnetic field pulse has a sinusoidal, rectangular and/or stochastic shape.

More preferred, the pulsed electromagnetic field has a frequency in the range of about 5 to 750 kHz, preferably of about 50 to 350 kHz, most preferably of about 250 kHz.

In a further embodiment, the pulsed electromagnetic field has a power in the range of about 10 to 200 Watt, preferably of about 20 to 100 Watt, most preferably of about 45 Watt. In another embodiment, the pulsed electromagnetic field has a magnetic field strength in the range of 100 to 150 µTesla.

Preferably, the exposing in step (g) is carried out for a time period of about 2 to 5 minutes.

More preferably, the exposing in step (g) is repeated, and preferably is carried out for three times.

In a further embodiment, the treatment is treatment in a subject.

Most preferred, the subject is a vertebrate, preferably a mammal, most preferably a human.

Preferably, the subject is not pregnant.

In one embodiment, the plant material derived from *Rosa sp,* is from *Rosa canina.*

In one embodiment, the plant material derived from *Rosa sp.* is a fruit.

In one embodiment, the plant material derived from *Urtica dioica* and/or *Tanacetum vulgare* is a leave and/or a small stem.

In one embodiment, the drying in step (b) is carried out at a temperature in the range of about 20 to 50°C, preferably of about 37 to 45°C, most preferably of about 42°C.

In one embodiment, the drying in step (b) is carried out for a time period of about 3 to 4 days.

In one embodiment, the organic solvent is ethanol, preferably of about 60 to 96 % (v/v), more preferably of about 80 to 96 % (by volume), most preferably of about 96 % (by volume).

In one embodiment, the incubating in step (d) is carried out for a time period in the range of about 20 to 40 days, preferably of about 22 to 38 days, most preferably of about 25 to 35 days. In one embodiment, the incubating in step (d) is carried out at a temperature in the range of about 20 to 50°C, preferably of about 37 to 45°C, most preferably of about 42°C.

Preferably, the treatment is post-surgical treatment of Recurrent Respiratory Papillomatosis.

In one embodiment, selenium is added to a concentration of free selenium in the range of about 1-100 mg/l, preferably of about 5-50 mg/l, most preferably of about 10-20 mg/l.

The object of the present invention is further solved by a pharmaceutical composition, comprising the herbal extract for use in the treatment of Recurrent Respiratory Papillomatosis according to the present invention.

In one embodiment, the pharmaceutical composition additionally comprises a pharmaceutically acceptable carrier.

In one embodiment, the pharmaceutical composition is formulated for oral administration.

The object of the present invention is further solved by a kit comprising the pharmaceutical composition according to the present invention.

The term "stochastic shape" comprises the meaning that the electromagnetic field pulse is in the form of a noise. Preferably, the electromagnetic field pulse is of rectangular shape and is combined with a sinusoidal wave inside. The "power" (Watt) of the pulsed electromagnetic field means e.g. effective power. The value of the "magnetic field strength" (Tesla) of the pulsed electromagnetic field indicates e.g. from peak to peak.

The term "pharmaceutical composition", as used herein, is intended to comprise the herbal extract of the present invention. Also considered is a pharmaceutical composition comprising at least one pharmaceutically active component of the herbal extract of the present invention and/or at least one derivative or analogon of said active component and corresponding salts thereof.

The pharmaceutical composition can be, for example, in a liquid form, e.g. a solution, syrup, elixier, emulsion and suspension, or in a solid form, e.g. a capsule, caplet, tablet, pill, powder, and suppository. Granules or semi-solid forms and gelcaps are also considered. In case that the pharmaceutical composition is a liquid or a powder, the dosage unit optionally is to be measured, e.g. in the dosage unit of a teaspoonful. In addition to the herbal extract or the pharmaceutically active component, the pharmaceutical composition can comprise, for example, flavouring agents, sweeteners, dyes, preservatives, stabilizers, colouring agents, diluents, suspending agents, granulating agents, lubricants, binders and disintegrating agents. A tablet, for example, can be coated. A liquid to be injected should be sterile. Also considered are transdermal delivery systems and liposomal systems. All of the formulations mentioned can be intended for immediate release, timed release and sustained release.

The term "pharmaceutically acceptable", as used herein, means at least non-toxic. The "pharmaceutically acceptable carrier", as meant in the present disclosure, may take a wide variety of forms depending upon the desired route of administration. The term comprises conventional pharmaceutical diluents such as water or ethanol and conventional tableting ingredients such as corn starch, lactose, sucrose, sorbitol, talc, stearic acid, magnesium stearate, dicalcium phosphate or gums.

Administration of the pharmaceutical composition of the present invention can use different routes, such as oral, sublingual, parenteral, intravenous, intraperitoneal, nasal, vaginal, rectal, subcutaneous, intradermal, intramuscular and topic. A dosage unit can be administered once or several times a day, week or month. The delivery can also be continuously by infusion or through a transdermal sustained release system, for example.

Thus, the present invention provides a combinatory herbal extract from *Rosa sp., Urtica dioica* and/or *Tanacetum vulgare* treated by electromagnetic field radiation. Clinical data showed a beneficial effect of the extract in the treatment of RRP. Studies conducted both *in vitro* and in experimental animal models revealed that the extract does not exert toxicity, mutagenicity, or oncogenicity. Pregnancy, however, is a contraindication.

### Detailed description of the invention

The invention will now be described in more detail by the following examples with the intention to exemplify the invention. The examples, however, are not intended to have any limiting effect on the subject-matter of the claims or on the scope of protection.

### EXAMPLE 1: Preparation of raw herbal extracts

Leaves and small stems of nettle (*Urtica dioica*) and tansy (*Tanacetum vulgare*) are collected from wild fields. After separation of useful parts and initial cleaning, the material is dried on a wooden network in a dark place for 3-4 days, preferably at 42°C. In dried condition, the plant material should be green without any change in colour, and leaves and stems should be brittle.

For extraction, airtight glass vessels are used. The dried plant material is broken into small pieces (2-5 cm) and placed into the glass vessels such that there is no space left. After packing (compressing), EtOH (96 %; herein, % of an ethanolic solution refers to % by volume)) is added until the vessel is filled completely. The vessels are placed into an incubator (37-45°C, preferably 42°C) for 20-40 days until a dark green solution appears.

For the extraction of wild rose (*Rosa canina*), dried fruits are used. In further embodiments, other species of *Rosa sp.* can be used alternatively or in addtion. The fruits are filled into airtight vessels up to a half and EtOH is added. The vessels are kept in an incubator (37-45°C, preferably 42°C) for 20-40 days until an orange-red coloured extract appears.

After the incubation period, when the plant material is colourless, the extracts are collected by separating them from plant material using a cloth filter.

### EXAMPLE 2: Electromagnetic treatment

The extract of *Rosa canina* is exposed to an electromagnetic field for 3 min. Then, 50-70 ml of the radiated *Rosa canine* extract is transferred to 2 1 of *Urtica dioica* and *Tanacetum vulgare* extracts, respectively. To each litre of the combined *Urtica*/*Rosa* and *Tanacetum*/*Rosa* extracts, respectively, 16 mg of selenium and 150 mg urea is added. In alternative embodiments, either selenium or urea is added. Then, the vessels are sealed again and kept in the incubator for 24 h at 42°C. After incubation, the vessels are exposed 4 times to an electromagnetic field, 3 min each, and are pooled together. The resulting extract is passed sequentially through a 5, 0.45 and 0.22 µm filter, respectively, and partitioned to sterile vials. After labelling and packaging, the herbals extract is ready for use.

The electromagnetic field, to which the raw extracts are exposed, is pulsed, powerful and monopolar in that the direction of the electric current generated in a Magnetic Impulse Generator (MIG) apparatus doesn't change. The pulsed magnetic field has a very high frequency ranging from 5 kHz-750 kHz. In this example, the pulse of a rectangular shape is used. Nevertheless, in other embodiments, a sinusoidal or stochastic shape is considered as well.

Preferably, the pulse is of rectangular shape and is combined with a sinusoidal wave inside. Although it is not intended to be bound to any theory, it is hypothesised that the special kind of the produced pulse causes some changes in the physical configuration of atoms in the molecules and/or arrangements of molecules thus leading to altered chemical properties.

In the preparation of the herbal extracts, 3-4 times radiation of electromagnetic pulses of high frequency is used for 2-5 min each. The electrical power (e.g. effective power) of the pulses is about 20 to 100 Watt, and the best effect is obtained at 45 Watt.

### EXAMPLE 3: Pre-clinical studies

Pre-clinical studies to investigate the drug's acute toxicity, chronic toxicity, mutagenicity, embryotoxicity and teratogenicity, effects on the reproductive function, and immuno-related effects, were done as described in details in prior art WO 2007/087825 Al.

In conclusion, the drug at the doses of 0.07 ml/kg and 0.18 ml/kg, did not show any serious side effects, and based on all of the experiments conducted and the obtained results, the herbal extract was recommended for clinical trials with the only contraindication of pregnancy.

### EXAMPLE 4: Pharmacologic effects related to RRP of the herbal extract

Nine newly infected and/or previously treated RRP patients with surgical intervention 3 times through one year, received the inventive herbal extract, also called IMOD. Patients were 2-56 years old. The medical protocol was categorized in two phases; 1. Intravenous administration of IMOD, 1.5 mg/kg for 10 days and then increased to 6 mg/kg for a six months period; 2. IMOD oral solution administration.

A complete blood chemistry profile, including blood cell count, was obtained for all patients before the start of study as well as 3 months post-treatment. These patients were followed for 12 months to evaluate the side effects and improvement in clinical signs. The results of treatment were estimated by decreasing the recurrence of symptoms and comparing pre- and post-treatment severity score.

Nine patients (4 males and 5 females, with the average age of 13.05 years) received IMOD along with surgical excision within two years. One of these patients was excluded from the study because of unwillingness to continue treatment.

**Table 1**

| **Baseline characteristics** | | | |
|---|---|---|---|
| | **Patients** | **Sex** | **Age** |
| | 4 | M | 4.87±4.17 |
| | 5 | F | 19.60±20.94 |
| **Total** | 9 | | |

Six patients had been previously treated by surgical excision from 1 to 5 times. In these patients improvement in clinical signs were detected after IMOD administration.

In 3 patients (supra-glott and glott lesions were diagnosed in 2/3 cases) the clinical score decreased to 2-3.

Neither in one patient suffering from lesions in lower airway tract nor in the 56-year-old, interventions was effective.

In 6/8 patients improvement in clinical signs were evaluated. IMOD administration was effective for 2 patients with glott and supra-glott lesions which clinical scoring decreased to 2-3. This finding is related to anatomical location of lesions, better post-surgical prognosis and lower disease severity.

Two patients did not show decreasing in severity scores which may be due to senile factors in one and lower respiratory tract involvement in another.

Based on existing observations, IMOD administration is an effective immunomudulatory drug for treatment of recurrent respiratory papillomatosis in human.

### EXAMPLE 5: Determination of the maximum tolerable dose of the herbal extract

This study was conducted to determine the maximum tolerable dose (MTD) of the herbal extract in HIV infected patients and its possible side-effects and toxicity that can cause dose limitation (dose limiting toxicities, DLTs).

The study protocol was based on the dose escalation method. The effects of the herbal extract on viral load and CD4 count of patients were evaluated as by-products. Four cohorts of patients (3 patients each) were selected and treated for 28 days (4 weeks) with escalated doses of the extract. A base dose of the extract has been determined according to LD₁₀ (10 % of the lethal dose) in former animal experiments. Patients were observed carefully for signs and symptoms of side-effects and toxicity by physical examination and laboratory workups according to the protocol.

All patients were male in the age of 28-60 years (mean: 41.6 years.). In the first cohort, the daily dose of 2 ml of extract in 100 ml warm normal saline was infused over 0.5-1 hr intravenously for 28 days. No toxicity or major side-effects were observed except for an increase in sweating and weight loss in 2 patients. In the second cohort, three other patients received a daily dose of 4 ml. There were no major side-effects and toxicity in this group. In the third cohort of originally 4 patients, one patient was excluded due to non-compliance and inability for regular daily attendance and the daily dose of 6.7 ml administered. In this group there were not only no major dose limiting toxicity and side-effects but also no minor ones. In the fourth cohort, three other patients received the daily dose of 10 ml, and there were no major side-effects and toxicity in this group too.

For the MTD study, a total of 12 patients were included in the study, who were treated for 4 weeks with escalated doses of the herbal extract. There was not toxicity or side-effects in all cohorts.

The features disclosed in the foregoing description and in the claims may both separately and in any combination thereof, be material for realizing the invention in diverse forms thereof.

## Claims

1. Herbal extract obtainable by a method, comprising the following steps:
(a) providing a plant material derived from *Rosa sp,, Urtica dioica* and/or *Tanacetum vulgare;*
(b) drying the plant material;
(c) adding an organic solvent;
(d) incubating the mixture of plant material and organic solvent;
(e) obtaining the herbal extract;
(f1) adding selenium and/or an organic or inorganic salt thereof; and/or
(f2) adding urea; and
(g) exposing the herbal extract to a pulsed electromagnetic field,
for use in the treatment of Recurrent Respiratory Papillomatosis.

2. Herbal extract for use in the treatment of Recurrent Respiratory Papillomatosis according to claim 1, wherein the electromagnetic field pulse has a sinusoidal, rectangular and/or stochastic shape.

3. Herbal extract for use in the treatment of Recurrent Respiratory Papillomatosis according to claim 1 or 2, wherein the pulsed electromagnetic field has a frequency in the range of about 5 to 750 kHz, preferably of about 50 to 350 kHz, most preferably of about 250 kHz.

4. Herbal extract for use in the treatment of Recurrent Respiratory Papillomatosis according to any of claims 1 to 3, wherein the pulsed electromagnetic field has a power in the range of about 10 to 200 Watt, preferably of about 20 to 100 Watt, most preferably of about 45 Watt.

5. Herbal extract for use in the treatment of Recurrent Respiratory Papillomatosis according to any of claims 1 to 4, wherein the pulsed electromagnetic field has a magnetic field strength in the range of 100 to 150 µTesla.

6. Herbal extract for use in the treatment of Recurrent Respiratory Papillomatosis according to any of claims 1 to 5, wherein the exposing in step (g) is carried out for a time period of about 2 to 5 minutes.

7. Herbal extract for use in the treatment of Recurrent Respiratory Papillomatosis according to any of claims 1 to 6, wherein the exposing in step (g) is repeated, and preferably is carried out for three times.

8. Herbal extract for use in the treatment of Recurrent Respiratory Papillomatosis according to any of claims 1 to 6, wherein treatment is treatment in a subject.

9. Herbal extract for use in the treatment of Recurrent Respiratory Papillomatosis according to claim 8, wherein the subject is a vertebrate, preferably a mammal, most preferably a human.

10. Herbal extract for use in the treatment of Recurrent Respiratory Papillomatosis according to claim 8 or 9, wherein the subject is not pregnant.

11. Pharmaceutical composition comprising a herbal extract according to any of the claims 1-10 and a pharmaceutically acceptable carrier.

12. Kit comprising the pharmaceutical composition according to claim 11.
